# EUROPEAN PATENT APPLICATION

(11) **EP 4 613 849 A1**
(43) Date of publication of application: **10.09.2025**
(21) Application number: 23885678.5
(22) Date of filing: 27.10.2023
(51) Int. Cl.: C12N 5/077

(54) **RENAL CELL AGGREGATE, AND METHOD FOR PRODUCING RENAL CELL AGGREGATE**

(30) Priority: 04.11.2022 JP 2022177567
(71) Applicant: Nikkiso Co., Ltd., Tokyo 150-6022 (JP)
(72) Inventor: TAKAHASHI, Etsushi, Kanazawa-shi, Ishikawa 920-0177 (JP)
(74) Representative: Hoffmann Eitle
(86) International application number: PCT/JP2023/038925
(87) International publication number: WO 2024/095922

(57) **Abstract**

Provided is a renal cell aggregate in which an expression level of a transporter gene is improved. One aspect of the present invention is a renal cell aggregate. The aggregate satisfies at least one or more of the following conditions:
(a) an expression level of OAT1 gene is 1 × 10⁻³ or more (GAPDH ratio);
(b) an expression level of OAT3 gene is 1 × 10⁻⁴ or more (GAPDH ratio);
(c) an expression level of OCT2 gene is 1 × 10⁻³ or more (GAPDH ratio);
(d) an expression level of MATE1 gene is 1 × 10⁻⁴ or more (GAPDH ratio);
(e) an expression level of MATE2 gene is 1 × 10⁻⁵ or more (GAPDH ratio);
(f) an expression level of MDR1 gene is 1 × 10⁻³ or more (GAPDH ratio); and
(g) an expression level of URAT1 gene is 1 × 10⁻⁶ or more (GAPDH ratio).

## Description

### Technical Field

The present invention relates to a renal cell aggregate and a method for producing the renal cell aggregate.

### Background Art

A drug administered to a living body is absorbed into the living body, and then excreted from blood into urine through a proximal tubule in the kidney. Therefore, renal damage is often caused by nephrotoxicity of the drug. In drug discovery research, it is very important to examine pharmacokinetics in the kidney in order to discover an action of a drug. For this reason, development of a drug discovery support device capable of evaluating pharmacokinetics and toxicity using renal cells is desired. In addition, such a drug discovery support device is also useful for development of a therapeutic agent for a disease related to the kidney (for example, diabetes, kidney cancer, or hyperuricemia).

A cell aggregate of renal cells has been proposed as what is useful for confirmation of pharmacokinetics and drug discovery (see Patent Literature 1).

### Citation List

### Patent Literature

Patent Literature 1: JP 2021-191305 A

### Summary of Invention

### Technical Problem

In a conventional renal cell aggregate, an expression level of a transporter gene necessary for evaluating pharmacokinetics and toxicity is insufficient, and there is room for development of a novel renal cell aggregate.

The present invention has been made in view of the above problem, and an object of the present invention is to provide a renal cell aggregate in which an expression level of a transporter gene is improved and a method for producing the renal cell aggregate.

### Solution to Problem

One aspect of the present invention is a renal cell aggregate. The renal cell aggregate satisfies at least one or more of the following conditions:
(a) an expression level of OAT1 gene is 1 × 10⁻³ or more (GAPDH ratio);
(b) an expression level of OAT3 gene is 1 × 10⁻⁴ or more (GAPDH ratio);
(c) an expression level of OCT2 gene is 1 × 10⁻³ or more (GAPDH ratio);
(d) an expression level of MATE1 gene is 1 × 10⁻⁴ or more (GAPDH ratio);
(e) an expression level of MATE2 gene is 1 × 10⁻⁵ or more (GAPDH ratio);
(f) an expression level of MDR1 gene is 1 × 10⁻³ or more (GAPDH ratio); and
(g) an expression level of URAT1 gene is 1 × 10⁻⁶ or more (GAPDH ratio).

The renal cell aggregate according to the above-described aspect may have a diameter of 150 µm or more and 350 µm or less. The number of renal cells constituting the aggregate may be 400 or more and 2100 or less. A cell passage number of the renal cells may be 3 or 4. A doubling time of the renal cells may be 20 to 36 hours.

Another aspect of the present invention is a method for producing a renal cell aggregate. The method for producing a renal cell aggregate includes: a pre-culture step of culturing renal cells under a condition that a doubling time of the renal cells after culture is 20 to 36 hours; an aggregate forming step of forming an aggregate of the renal cells that have been subjected to the pre-culture step; and a standing step of allowing the formed aggregate to stand for 24 hours or more.

In the pre-culture step in the method for producing a renal cell aggregate according to the above-described aspect, the renal cells may be cultured at a seeding density of 2.1 × 10³ to 4.4 × 10³ cells/cm² for 48 to 120 hours.

### Advantageous Effects of Invention

According to the present invention, it is possible to provide a technique related to a renal cell aggregate in which an expression level of a transporter gene is improved.

### Brief Description of Drawings

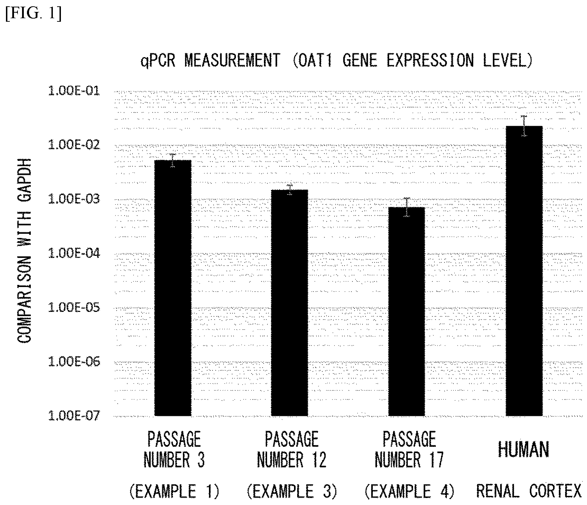

[Fig. 1] Fig. 1 is a graph illustrating expression levels of OAT1 genes (GAPDH ratio) of Examples 1 to 4 and human renal cortex.

### Description of Embodiments

Hereinafter, embodiments of the present invention will be described in detail. Note that, in the present specification, the notation "a to b" in description of a numerical range represents a or more and b or less unless otherwise specified.

### (Renal cell aggregate)

A renal cell used in the present disclosure may be any renal cell as long as it can be cultured, and a source thereof is not limited. The renal cell is preferably derived from a mammal, and is preferably derived from primates such as humans and monkeys. In addition, the renal cell may be derived from a normal kidney or a kidney having a disease depending on a purpose. Examples of the renal cell include cells constituting epithelium, cortex, proximal tubule, distal tubule, collecting duct, and glomerulus, and specific examples thereof include a proximal tubule epithelial cell (RPTEC), and a mesangial cell. The renal cell may be a renal cell derived from a stem cell such as an iPS cell or an ES cell. In addition, the renal cell may be an immortalized renal cell, an established cell (an HK-2 cell or the like), a cell derived from other animal species (an MDCK cell, an LLC-PK1 cell, a JTC-12 cell, or the like), or a forcibly expressed cell obtained by introducing a gene into a renal cell in order to express a protein such as a specific transporter. More specific examples of the renal cell include a human proximal tubule epithelial cell, a human distal tubule epithelial cell, and a human collecting duct epithelial cell collected and isolated from the kidney, and a proximal tubule epithelial cell, a distal tubule epithelial cell, and a collecting duct epithelial cell induced to differentiate from a human iPS cell or a human ES cell. For use in drug discovery research, a proximal tubule epithelial cell is preferable, and a proximal tubule epithelial cell derived from a human normal kidney is particularly preferable.

Here, the "aggregate" of renal cells refers to a massive aggregate of several or more cells. The aggregate is also referred to as an aggregated mass or a spheroid.

A renal cell aggregate (hereinafter, the renal cell aggregate may be simply referred to as an "aggregate") according to an embodiment satisfies at least one or more of the following conditions.
(a) an expression level of OAT1 gene is 1 × 10⁻³ or more (GAPDH ratio);
(b) an expression level of OAT3 gene is 1 × 10⁻⁴ or more (GAPDH ratio);
(c) an expression level of OCT2 gene is 1 × 10⁻³ or more (GAPDH ratio);
(d) an expression level of MATE1 gene is 1 × 10⁻⁴ or more (GAPDH ratio);
(e) an expression level of MATE2 gene is 1 × 10⁻⁵ or more (GAPDH ratio);
(f) an expression level of MDR1 gene is 1 × 10⁻³ or more (GAPDH ratio); and
(g) an expression level of URAT1 gene is 1 × 10⁻⁶ or more (GAPDH ratio).

An expression level of each transporter gene in a renal cell culture can be measured by a general real-time PCR method (qPCR method). The "GAPDH ratio" is a value obtained by correcting an expression level of each transporter gene by GAPDH that is a housekeeping gene, and is obtained by dividing the expression level of each transporter gene by an expression level of a GAPDH gene. By calculating the expression level as a "GAPDH ratio", a relative gene expression level of each transporter can be measured regardless of a difference in the number of collected cells. The GAPDH ratio is a value obtained by correcting an error between samples at the time of sample preparation in the qPCR method.

OAT1 (Organic Anion Transporter 1), OAT3 (Organic Anion Transporter 3), OCT2 (Organic Cation Transporter 2), MATE1 (Multidrug And Toxin Extrusion 1), MATE2 (Multidrug And Toxin Extrusion 2), and MDR1 (Multiple Drug Resistance 1) are genes encoding a protein involved in drug transport.

URAT1 (Urate Transporter 1) is a gene encoding a protein involved in resorption of uric acid.

When the expression level of OAT1 gene is 1 × 10⁻³ or more (GAPDH ratio) (satisfies condition (a)), Tenofovir induced toxicity, which is toxicity via OAT1, is likely to be exhibited, and a renal cell aggregate suitable for evaluation of Tenofovir induced toxicity can be obtained.

When the expression level of OAT3 gene is 1 × 10⁻⁴ or more (GAPDH ratio) (satisfies condition (b)), drug uptake and induction of toxicity via OAT3 are likely to be exhibited. OAT3 uses a compound having a larger molecular weight and higher lipid solubility than OAT1 as a substrate, and thus has an effect of enabling uptake of a wider range of drugs.

When the expression level of OCT2 gene is 1 × 10⁻³ or more (GAPDH ratio) (satisfies condition (c)), Cisplatin induced toxicity, which is toxicity via OCT2, is likely to be exhibited, and a renal cell aggregate suitable for evaluation of Cisplatin induced toxicity can be obtained.

When the expression level of MATE1 gene is 1 × 10⁻⁴ or more (GAPDH ratio) (satisfies condition (d)), a renal cell aggregate having an excretion function of a drug taken into blood in the kidney into urine can be obtained.

When the expression level of MATE2 gene is 1 × 10⁻⁵ or more (GAPDH ratio) (satisfies condition (e)), a renal cell aggregate having an excretion function of a drug taken into the kidney into urine can be obtained. By simultaneous expression of MATE2 gene with MATE1, a renal cell aggregate having an excretion function of a wide range of drugs into urine can be obtained.

When the expression level of MDR1 gene is 1 × 10⁻³ or more (GAPDH ratio) (satisfies condition (f)), a renal cell aggregate having an excretion function of a drug taken into the kidney into urine can be obtained. Since MDR1 gene is a transporter having wide substrate recognizability, a renal cell aggregate having a function of excreting various types of drugs can be obtained.

When the expression level of URAT1 gene is 1 × 10⁻⁶ or more (GAPDH ratio) (satisfies condition (g)), a renal cell aggregate having a function of reabsorbing uric acid can be obtained.

The renal cell aggregate according to the embodiment preferably satisfies two conditions, three conditions, four conditions, five conditions, six conditions, or seven (all) conditions among the conditions described above. When two or more conditions are satisfied, at least condition (a) and condition (f) are more preferably satisfied.

OAT1 is a transporter responsible for uptake of a drug from the inside of a blood vessel after drug administration. Meanwhile, MDR1 is a transporter that excretes a drug that has been taken into a ureter side. Therefore, by satisfying conditions (a) and (f), nephrotoxicity or renal pharmacokinetics in drug discovery can be appropriately evaluated.

A ratio R between the gene expression level of OAT1 and the gene expression level of MDR1 (gene expression level of OAT1/gene expression level of MDR1) is preferably 0.01 or more, more preferably 0.1 or more, still more preferably 0.5 or more.

When the ratio R is equal to or more than the above-described lower limit value and as the ratio R approaches "1", uptake and excretion of a drug in the kidney are more normally performed, a human renal cell having a similar function to the kidney in a human living body is obtained, and a cell in which nephrotoxicity or renal pharmacokinetics in drug discovery can be appropriately evaluated is obtained.

In addition, when the ratio R is equal to or more than the above-described lower limit value and as the ratio R approaches "1", it is possible to have a function similar to that of a normal kidney that excretes a drug taken in from a blood vessel to a ureter side.

The diameter of the aggregate is preferably 150 µm or more and 350 µm or less, more preferably 200 µm or more and 350 µm or less, and still more preferably 250 µm or more and 350 µm or less.

The number of renal cells constituting the aggregate is preferably 400 or more and 2100 or less, and more preferably 900 or more and 1600 or less. When the number of renal cells constituting the aggregate is within the above range, higher homogeneity among the plurality of aggregates is possible.

The cell passage number of the renal cells used in the pre-culture step is preferably 3 or 4. By setting the cell passage number to 3 or 4, a renal cell aggregate in which a metabolic function or the like is activated can be prepared, and an aggregate satisfying at least one of conditions (a) to (g) described above can be obtained.

Since many human somatic cells do not proliferate indefinitely, when human renal cells are also cultured in vitro, the cells do not proliferate after a certain number of passages. In a chromosome of a cell, there is a repetitive sequence called a telomere, and the telomere is shortened by cell proliferation to cause aging of the cell. Since aging of a cell is also caused by an external environmental factor such as oxidative stress, aging of a cell is observed in an in vitro culture state that is ex vivo. When a cell is aged, DNA is damaged, and it is difficult to shift to a proliferation cycle in a cell cycle. Therefore, it is considered that, even in the renal cells, the cells are aged by repeated passages, and proliferation capability decreases (doubling time increases). In addition, when aging of a cell occurs, a decrease in protein expression and expression of abnormal protein due to DNA damage are induced, and thus it is considered that the function itself of increasing the expression of the drug transporter decreases even when an aggregate is formed using aged renal cells.

From these, a renal cell aggregate having high expression of a drug transporter can be obtained by forming an aggregate using non-aged renal cells having the passage number of 3 or 4. In addition, since it is difficult to collect the number of cells necessary for preparing an aggregate in renal cells having reduced proliferation capability, a sufficient amount of renal cell aggregates can be prepared by using renal cells in a proliferation cycle having a doubling time of 20 to 36 hours.

The roundness of the aggregate is preferably 0.3 or more and 1.0 or less, and more preferably 0.4 or more and 1.0 or less. The aspect ratio of the aggregate is preferably 1.0 or more and 2.0 or less, and more preferably 1.0 or more and 1.5 or less. Note that the roundness and the aspect ratio of the aggregate can be calculated by recognizing one aggregate in an image {slice image (cross-sectional view)} with a CQ1 (confocal image cytometer, manufactured by Yokogawa Electric Corporation) on the basis of a well-known method.

### (Method for producing renal cell aggregate)

A method for producing a renal cell aggregate according to an embodiment includes a pre-culture step, an aggregate forming step, and a standing step. Hereinafter, details of each step will be described.

### (Pre-culture step)

In the pre-culture step, renal cells are cultured on a culture vessel such as a culture dish under a condition that the doubling time of the renal cells after culture is 20 to 36 hours, preferably 24 to 32 hours. Examples of specific pre-culture conditions include a renal cell seeding density of 2.1 × 10³ to 4.4 × 10³ cells/cm² and a culture time of 48 to 120 hours. A cell confluence at this time is 50 to 80%.

As the medium, any known medium can be appropriately used. For example, in a case of culture of proximal tubule epithelial cells, a commercially available tubular cell culture medium can be used, and preferable examples thereof include REGM (registered trademark) (LONZA), EpiCM (registered trademark) (ScienCell), and KeratinocyteSFM (registered trademark) (Thermo Fisher Scientific).

By performing pre-culture such that the doubling time of the renal cells after culture is 20 to 36 hours, proliferation capability of the renal cells is brought into the most activated state, a renal cell aggregate in which a metabolic function is activated can be prepared, and an aggregate satisfying at least one of conditions (a) to (g) described above can be obtained.

### (Aggregate forming step)

In the aggregate forming step, an aggregate of renal cells subjected to the pre-culture step is formed.

By using a medium and a culture vessel suitable for cells to be cultured, renal cells can be cultured in the aggregate forming step according to a conventional method, for example, under conditions of 37°C and 5%CO₂. The culture may be any of static culture, shaking culture, stirring culture, and the like. The culture may be adhesion culture, but it is preferable to perform the culture in a low adhesion state (for example, suspension culture) for at least a part of a period. The renal cells are cultured in a low adhesion state in a culture vessel, whereby an aggregate can be formed. The "low adhesion state" refers to a state in which all or most of the cells are not adhering to a surface of the culture vessel, and includes a state in which all or most of the cells are separated from the surface of the culture vessel and a state in which even when the cells are in contact with the surface of the culture vessel, the cells can be easily separated from the surface of the culture vessel by coating of the culture vessel, convection of the medium, or the like without using a tool, an enzyme, or the like.

For example, in some cases, a renal cell aggregate is formed within 24 hours after initiation of culture of the renal cells. Then, by culturing the renal cells in an aggregate state for a part of a period, it is possible to restore a physiological function of the renal cells, the physiological function having been lowered by dedifferentiation. A period during which the renal cells are cultured in a state of low adhesion to the culture vessel is generally desirably 120 hours or more. This makes it possible to obtain cultured renal cells in a state of higher expression of the physiological function. During the culture period, the medium is preferably replaced periodically. For example, the medium is replaced every two days.

As the medium, any known medium can be appropriately used. For example, in a case of culture of proximal tubule epithelial cells, a commercially available tubular cell medium can be used, and preferable examples thereof include REGM (registered trademark) (LONZA), EpiCM (registered trademark) (ScienCell), and KeratinocyteSFM (registered trademark) (Thermo Fisher Scientific).

In addition, conventionally known materials and additives useful for cell culture can be appropriately used. For example, collagen I (type I collagen) can be added to the medium. Collagen I has an action of causing the renal cells to adhere to each other. Therefore, formation of an aggregate is promoted by culturing the renal cells in a medium containing collagen I. Collagen I is preferably full-length collagen I, but may be an α1 chain or an α2 chain constituting collagen I, or a collagen peptide obtained by fragmenting chains. A source of collagen I is not particularly limited, and collagen I may be derived from humans or other animals.

Any culture vessel can be used. Here, in order to promote formation of an aggregate, the culture vessel is preferably subjected to a cell non-(low) adhesion treatment or is preferably made of a cell non-(low) adhesion material. Examples of the cell non-(low) adhesion treatment include a cell low adhesion hydrogel coating treatment, a 2-methacryloyloxyethyl phosphorylcholine (MPC) coating treatment, a ProteoSave (registered trademark) SS coating treatment, and a mirror polishing treatment to a surface of the vessel. Examples of the cell non-(low) adhesion material include glass, and polymer materials such as a low-density polyethylene, a medium-density polyethylene, polyvinyl chloride, a polyethylene-vinyl acetate copolymer, a poly(ethylene-ethyl acrylate) copolymer, a poly(ethylene-methacrylate) copolymer, a poly(ethylene vinyl acetate) copolymer, and mixtures of two or more of these polymers.

When a large amount of aggregates are formed, a high-density spheroid preparing plate or a high-density spheroid preparing dish can be used. Furthermore, a culture vessel such as a spinner flask may be used as necessary. For example, it is preferable to use a culture vessel of ELPLASIA (registered trademark) series (Corning), a culture vessel of EZSPHERE (registered trademark) series (AGC Techno Glass Co., Ltd.), or the like. These examples of the culture vessel include types such as a 6-well plate, a 24-well plate, a 96-well plate, a 384-well plate, and dishes of various sizes, and the number of aggregates that can be prepared varies depending on the size of a bottom area of the vessel. For example, when a 96-well plate (V bottom), a 96-well plate (U bottom), or a 384-well plate (U bottom) subjected to a low adhesion treatment is used, one aggregate is formed in one well.

An aggregate prepared using a high-density spheroid preparing plate, a high-density spheroid preparing dish, or the like can be collected and cultured while being suspension-shaken. In a case where an aggregate is cultured while being suspension-shaken, it is preferable to culture the aggregate by placing a dish, a plate, or the like subjected to a cell non-(low) adhesion treatment on a shaker. As the shaker, a reciprocating shaker or a swirling shaker can be used.

### (Standing step)

In the standing step, the renal cell aggregate obtained in the aggregate forming step is seeded, and then the aggregate is preferably allowed to stand for 24 hours or more, and more preferably allowed to stand for 48 hours or more, for example, under conditions of 37°C and 5%CO₂. An upper limit of the standing time is the number of days during which an effect of standing is obtained, and the number of days during which supply of a medium (nutrients) to the cells is not insufficient is preferable. For example, the standing time is preferably five days or less, and more preferably four days or less. While the renal cells are allowed to stand, the medium is not replaced. By allowing the obtained aggregate to stand for the above period, adhesion between cells in the aggregate can be further strengthened. In addition, a spherical human renal cell aggregate can be formed in the culture process without applying a load such as a shear stress to the renal cells during aggregate formation. In other words, the renal cell aggregate is subjected to the standing step, whereby the roundness and/or the aspect ratio are within the above-described ranges, and approach 1. As a result, it is presumed that the expression level of each of the above-described transporter genes is increased. A period during which the roundness and the aspect ratio approach 1 is the standing step, and after the standing step, the medium is replaced at a frequency of once every two days, and removal of waste products in the medium and supply of nutrients are periodically performed.

### (Use of renal cell aggregate)

In the renal cell aggregate of the present embodiment, the expression level of each of the above-described transporter genes is increased, and therefore the renal cell aggregate can be provided as a renal cell product for a drug evaluation system. Examples of the drug evaluation system include a system for evaluating pharmacokinetics in renal cells and nephrotoxicity in drug discovery. In addition, such renal cells can also be used as a tool for mechanism analysis of diseases related to the kidney, such as diabetes, kidney cancer, and hyperuricemia, and therapeutic agent search.

The embodiments of the present invention have been described above, and these are examples of the present invention, and various configurations other than the above can be adopted.

### Examples

Hereinafter, the present invention will be described with reference to Examples and Comparative Example, but the present invention is not limited thereto.

### (Example 1)

### <Production of renal cell aggregate>

Human proximal tubule epithelial cells {Clonetics (registered trademark), Catalog No. CC-2553, RPTEC-kidney proximal tubule epithelial cells} obtained from LONZA were used as renal cells. A cryovial stored in a liquid nitrogen storage was immersed in a thermostat at 37°C for thawing. After thawing, a cell suspension in the cryovial was mixed with a recommended medium {REGM (registered trademark), LONZA}, and the pre-culture step was performed in a culture dish. As conditions at the time of the pre-culture, a seeding density was 4.2 × 10³ cells/cm², a culture time was 72 hours, and a passage number was 3. The pre-culture resulted in approximately 70% cell confluence. The renal cells subjected to the pre-culture step were collected, and a doubling time was confirmed on the basis of the number of cells before seeding and the number of cells at the time of collection, and found to be 27.5 hours.

The renal cells subjected to the pre-culture step were collected, and were subjected to the aggregate forming step. Specifically, the renal cells were cultured under conditions of 37°C and 5%CO₂ while the medium was replaced at a frequency of once every two days. The cells were collected before becoming confluent, and seeded on a 96-well V-bottom plate {PrimeSurface (registered trademark) plate 96 V, Sumitomo Bakelite Co., Ltd.} subjected to a cell low adhesion treatment such that the number of cells was 1000 per well, and cultured to form an aggregate. For two days (48 hours) after the aggregate formation, the culture plate was allowed to stand in an incubator as the standing step without performing a culture operation such as medium replacement. After the standing step, the aggregate was cultured for 240 hours or more while the medium was replaced at a frequency of once every two days. Noted that "confluent" means that a ratio of an area occupied by the cells to the entire culture surface of the culture vessel is about 100%, that is, means a state in which the cells have proliferated to the culture surface without gaps.

### (Example 2)

A renal cell aggregate was prepared in a similar manner to that in Example 1 except that the passage number was 9.

### (Example 3)

A renal cell aggregate was prepared in a similar manner to that in Example 1 except that the passage number was 12.

### (Example 4)

A renal cell aggregate was prepared in a similar manner to that in Example 1 except that the passage number was 17.

The following evaluation or measurement was performed for each of the renal cell aggregates of Examples 1 to 4.

### <Measurement of transporter gene expression level>

From each aggregate after standing for two days, mRNA was extracted and purified using RNeasy (registered trademark) Mini Kit (QIAGEN). Furthermore, cDNA was synthesized from this mRNA using QuantiTect (registered trademark) Whole Transcriptome Kit (QIAGEN). Using the cDNA as a template, expression levels of genes of GAPDH, OAT1, OAT3, OCT2, MATE1, MATE2, MDR1, and URAT1 were measured by a real-time PCR method using Thermal Cycler Dice (registered trademark) Real Time System 1 (Takara Bio Inc.). Note that, for all experiments, each sample was measured with n = 3 in one experiment. Table 1 presents primer names and oligo sequences of each gene used in the real-time PCR method.

**[Table 1]**

| Target gene | Primer name (Oligo name) | Oligo sequence (5' to 3' ) |
|---|---|---|
| hGAPDH | q-hGAPDII-F | TTGACGCTGGGGCTGGCATT |
| | q-hGAPDH-R | GTGCTCTTGCTGGGGCTGGT |
| hOAT1 | q-hOAT1-F | CTGTATCCCACAATGATCCG |
| | q-hOAT1-R | GGCAGTCATGCTCACCAGT |
| hOAT3 | q-hOAT3-F | GTCCATACGCTGGTGGTCTT |
| | q-hOAT3-R | GCTOAGCCTTTCTCCCTCTT |
| hOCT2 | q-hOCT2-F | GAAGCAACAGCACCAGACCT |
| | q-hOCT2-R | CCTGGTCTACCGGCTCACTA |
| hMATE1 | q-hMATE1-F | GCAACCACACTTGGAGTGATGG |
| | q-hMATE1-R | GAGCAGAATTCCCACTCCGAG |
| hMATE2 | q-hMATE2-F | GCAGGGCCAGTTTTCATTTA |
| | q-hMATE2-R | TGGGAGATGATGTTGGCATA |
| hMDR1 | q-hMDR1-F | GCCCTTGTTAGACAGCCTCATATTT |
| | q-hMDR1-R | GGACAGGCGGTGAGCAAT |
| hURAT1 | q-hURAT1-F | CCTTTCCGTTGATGGCAG |
| | q-hURAT1-R | GTCCCCTTCTTCCTCTGCTT |

An expression level of each obtained transporter gene was divided by an expression level of GAPDH gene to obtain an expression level (GAPDH ratio) of each transporter gene. Table 2 presents the calculation result of the expression level (GAPDH ratios) of each transporter gene.

(Diameter, roundness, and aspect ratio of aggregate)

Regarding the state of the obtained renal cell aggregate, the diameter, roundness, and aspect ratio of the aggregate were calculated by recognizing one aggregate in an image {slice image (cross-sectional view)} with a CQ1 (confocal image cytometer, manufactured by Yokogawa Electric Corporation) on the basis of a well-known method. The results obtained for the diameter, roundness, and aspect ratio of renal cell aggregate are presented in Table.

**[Table 2]**

| | Example 1 | Example 2 | Example 3 | Example 4 |
|---|---|---|---|---|
| Passage number | 3 | 9 | 12 | 17 |
| Seeding density (cells/cm²) | 4.2 × 10³ | 2.1 × 10³ | 2.1 × 10³ | 2.1 × 10³ |
| Culture period (hours) | 72 | 120 | 168 | 168 |
| Cell confluence (%) | 80 | 80 | 70 | 60 |
| Doubling time (hours) | 27.5 | 107 | 129 | 168 |
| Standing time after aggregate formation (hours) | 48 | 48 | 48 | 48 |
| OAT1 gene (GAPDH ratio) | 3.65 × 10⁻³ | - | 1.49 × 10⁻³ | 7.18 × 10⁻⁴ |
| OAT3 gene (GAPDH ratio) | 4.23 × 10⁻³ | - | 8.23 × 10⁻⁴ | 1.35 × 10⁻³ |
| OCT2 gene (GAPDH ratio) | 2.34 × 10⁻² | - | 4.11 × 10⁻³ | 4.88 × 10⁻³ |
| MATE1 gene (GAPDH ratio) | 3.84 × 10⁻³ | - | 2.10 × 10⁻³ | 1.52 × 10⁻² |
| MATE2 gene (GAPDH ratio) | 1,50 × 10⁴ | - | 1.59 × 10⁻⁴ | 0 |
| MDR1 gene (GAPDH ratio) | 9.80 × 10⁻³ | - | 1.21 × 10⁻² | 8.29 × 10⁻² |
| URAT1 gene (GAPDH ratio) | 1.55 × 10⁻⁵ | - | 6.33 × 10⁻³ | 0 |
| Diameter of aggregate (µm) | 322 | - | - | - |
| Roundness | 0.81 | - | - | - |
| Aspect ratio | 1.04 | - | - | - |

In Table 2 above, the item "-" indicates that it has not been performed.

In addition, an expression level of OAT1 gene (GAPDH ratio) was measured for human proximal tubule epithelial cells used for formation of the renal cell aggregate. Fig. 1 illustrates a graph of expression levels of OAT1 genes (GAPDH ratio) of Examples 1, 3, and 4 and human renal cortex.

### (Change in cell form and gene expression level in aggregate forming step)

In accordance with the above-described production of a renal cell aggregate, when the number of days from start of aggregate formation was 0 (immediately after seeding), 1 day, 2 days, and 11 days, the diameter, roundness, aspect ratio, and OAT1 expression level of the aggregate were calculated. It was confirmed that the expression level of OAT1 gene increased as the roundness and the aspect ratio approached 1.

**[Table 3]**

| Number of days after seeding | day0 | day1 | day2 | day11 |
|---|---|---|---|---|
| Diameter of aggregate (µm) | - | 294 | 266 | 322 |
| Roundness of aggregate cross section | - | 0.61 | 0.69 | 0.81 |
| Aspect ratio of aggregate cross section | - | 1.40 | 1.20 | 1.04 |
| OAT1 expression level (GAPDH ratio) | Less than 1.00 × 10⁻⁷ | Less than 1.00 × 10⁻⁷ | 1.10 × 10⁻⁶ | 3.65 × 10⁻³ |

### Industrial Applicability

In the renal cell aggregate of the present embodiment, the expression level of each of the above-described transporter genes is increased, and therefore the renal cell aggregate can be used as a renal cell product for a drug evaluation system. In addition, the renal cells of the present embodiment can also be used as a tool for mechanism analysis of diseases related to the kidney, such as diabetes, kidney cancer, and hyperuricemia, and therapeutic agent search.

## Claims

1. A renal cell aggregate satisfying at least one of the following conditions:
(a) an expression level of OAT1 gene is 1 × 10⁻³ or more (GAPDH ratio);
(b) an expression level of OAT3 gene is 1 × 10⁻⁴ or more (GAPDH ratio);
(c) an expression level of OCT2 gene is 1 × 10⁻³ or more (GAPDH ratio);
(d) an expression level of MATE1 gene is 1 × 10⁻⁴ or more (GAPDH ratio);
(e) an expression level of MATE2 gene is 1 × 10⁻⁵ or more (GAPDH ratio);
(f) an expression level of MDR1 gene is 1 × 10⁻³ or more (GAPDH ratio); and
(g) an expression level of URAT1 gene is 1 × 10⁻⁶ or more (GAPDH ratio).

2. The renal cell aggregate according to claim 1, wherein the aggregate has a diameter of 150 µm or more and 350 µm or less.

3. The renal cell aggregate according to claim 1 or 2, wherein the number of renal cells constituting the aggregate is 400 or more and 2100 or less.

4. The renal cell aggregate according to claim 1 or 2, wherein a cell passage number of the renal cells is 3 or 4.

5. The renal cell aggregate according to claim 1 or 2, wherein a doubling time of the renal cells is 20 to 36 hours.

6. A method for producing a renal cell aggregate, comprising:
a pre-culture step of culturing renal cells under a condition that a doubling time of the renal cells after culture is 20 to 36 hours;
an aggregate forming step of forming an aggregate of the renal cells that have been subjected to the pre-culture step; and
a standing step of allowing the formed aggregate to stand for 24 hours or more.

7. The method for producing a renal cell aggregate according to claim 6, wherein
in the pre-culture step, the renal cells are cultured at a seeding density of 2.1 × 10³ to 4.4 × 10³ cells/cm² for 48 to 120 hours.
